# EUROPÄISCHE PATENTANMELDUNG

(11) **EP 3 441 781 A1**
(43) Veröffentlichungstag der Anmeldung: **13.02.2019**
(21) Anmeldenummer: 17185859.0
(22) Anmeldetag: 11.08.2017
(51) Int. Cl.: G01R 33/483, G01R 33/561, G01R 33/565

(54) **BESCHLEUNIGTE MAGNETRESONANZ-MESSUNG**

(71) Anmelder: Siemens Healthcare GmbH, 91052 Erlangen (DE)
(72) Erfinder: Carinci, Flavio, 91052 Erlangen (DE); Zeller, Mario, 91054 Erlangen (DE)

(57) **Zusammenfassung**

Ein erfindungsgemäßes Verfahren zum Erstellen von Magnetresonanzdaten von mindestens zwei simultan manipulierten, sich nicht überschneidenden Schichten eines Untersuchungsobjekts mittels einer parallelen Akquisitionstechnik erreicht durch die Aufnahme von Referenzdaten derart, dass zwischen der Aufnahme von Schichten-Messdaten eines Schichten-Messdatensatzes, in dem die Messdaten aller simultan manipulierter Schichten überlagert enthalten sind, und seiner zugehörigen Referenzdaten keine Schichten-Messdaten eines anderen Schichten-Messdatensatzes aufgenommen werden, eine hohe Robustheit gegenüber Bewegungen des Untersuchungsobjekts.

## Beschreibung

Die Erfindung betrifft eine beschleunigte Magnetresonanz-Messung, insbesondere ein mit Schicht-Multiplexing beschleunigtes Single-Shot-Verfahren.

Die Magnetresonanz-Technik (im Folgenden steht die Abkürzung MR für Magnetresonanz) ist eine bekannte Technik, mit der Bilder vom Inneren eines Untersuchungsobjektes erzeugt werden können. Vereinfacht ausgedrückt wird hierzu das Untersuchungsobjekt in einem Magnetresonanzgerät in einem vergleichsweise starken statischen, homogenen Grundmagnetfeld, auch B₀-Feld genannt, mit Feldstärken von 0,2 Tesla bis 7 Tesla und mehr positioniert, so dass sich dessen Kernspins entlang des Grundmagnetfeldes orientieren. Zum Auslösen von Kernspinresonanzen werden hochfrequente Anregungspulse (RF-Pulse) in das Untersuchungsobjekt eingestrahlt, die ausgelösten Kernspinresonanzen als Echosignale gemessen, die als sogenannte k-Raumdaten gespeichert werden, und auf deren Basis, z.B. mittels einer mehrdimensionalen Fourier-Transformation, MR-Bilder rekonstruiert (oder Spektroskopiedaten ermittelt). Zur Ortskodierung der Messdaten werden dem Grundmagnetfeld in der Regel schnell geschaltete magnetische Gradientenfelder überlagert.

Dabei gibt es grundsätzlich zwei Arten nach einer Anregung der Kernspins Echosignale zu erzeugen. Einerseits können die angeregten Kernspins durch Schalten von Dephasierungs- und Rephasierungsgradienten so manipuliert werden, dass das Signal schneller zerfällt als dem dem gemessenen Gewebe inhärenten T2*-Zerfall geschuldet, sich aber nach einer gewissen Zeit, der Echozeit (TE), nach dem verwendeten RF-Anregungspuls ein sogenanntes zu messendes Gradientenecho (GRE) bildet. Derartige Sequenzen werden üblicherweise als GRE-Sequenzen bezeichnet. Andererseits kann auch durch Einstrahlen mindestens eines RF-Refokussierungspulses nach dem Einstrahlen eines RF-Anregungspulses nach einer wiederum Echozeit genannten Zeit nach dem RF-Anregungspuls ein sogenanntes Spinecho (SE) erzeugt werden, das gemessen wird und dessen Amplitude jedoch gemäß der dem gemessenen Gewebe inhärenten T2-Zerfall reduziert ist. Derartige Sequenzen werden üblicherweise als SE-Sequenzen bezeichnet. Es gibt auch Sequenzen, die sowohl Gradientenechos als auch Spinechos erzeugen, z.B. (true)FISP-Sequenzen. In jedem Fall werden die Anregung und die Messung der erzeugten Echosignale bei jeder Sequenz gegebenenfalls so lange wiederholt (z.B. unter Schaltung verschiedener Gradienten zur Ortskodierung) bis die gewünschte Anzahl an Echosignalen gemessen und im k-Raum gespeichert wurde, um das Untersuchungsobjekt abbilden zu können.

Unter den SE-Sequenzen sind insbesondere die TSE-Sequenzen (TSE: "Turbo Spin Echo"), welche auch unter den Namen FSE-("Fast Spin Echo") oder RARE- ("Rapid Acquisition with Refocussed Echoes") Sequenzen bekannt sind, weit verbreitet in der klinischen Anwendung. Der Vorteil der TSE-Sequenzen gegenüber der "einfachen" SE-Sequenz ist, dass nach einem RF-Anregungspuls mehrere Refokussierungspulse geschaltet werden, und dass dadurch auch mehrere Spinechosignale erzeugt werden. Dadurch wird die Datenaufnahme beschleunigt.

Bei sogenannten "Single-Shot" Verfahren können hierbei die gesamten aufzunehmenden k-Raumdaten, z.B. zur Abbildung einer abzubildenden Schicht eines Untersuchungsobjekts, nach nur einer RF-Anregung durch einen RF-Anregungspuls aufgenommen werden.

Ein Beispiel für eine solche Single-Shot TSE-Sequenz ist die HASTE-Sequenz ("Half-Fourier Acquisition Single-shot Turbo spin Echo imaging"), bei der zur Reduktion der aufzunehmenden k-Raumdaten zusätzlich ein "partial Fourier"-Verfahren, insbesondere das Half-Fourier-Verfahren eingesetzt wird. Dabei wird die Symmetrie des k-Raums gegenüber komplexer Konjugation genutzt, um nicht gemessene k-Raumdaten aus den gemessenen k-Raumdaten abzuleiten. Damit können nach nur einem Anregungspuls alle für das Verfahren nötigen k-Raumdaten einer abzubildenden Schicht aufgenommen werden. Sollen Schichten eines Untersuchungsobjekts gemessen werden, können z.B. alle benötigten k-Raumdaten einer Schicht mittels HASTE nach nur einer Anregung aufgenommen werden. Daher werden HASTE-Techniken üblicherweise für Aufnahmen des Thorax oder des Abdomens eingesetzt, wo sie die Abdeckung relativ großer interessierender Volumen ("volume of interest", VOI) innerhalb einer oder mehrerer Atemanhaltephasen mit einer reduzierten Sensitivität gegenüber physiologischer Bewegungen des Untersuchungsobjekts erlauben.

HASTE-Aufnahmetechniken sind unter anderem auch unter den Akronymen SS-FSE (Single-Shot fast spin echo), SSH-TSE (Single-SHot turbo spin echo), UFSE (ultra-fast spin echo), Single-Shot fast SE oder FASE bzw. Super-FASE (Fast Advanced Spin Echo) bekannt.

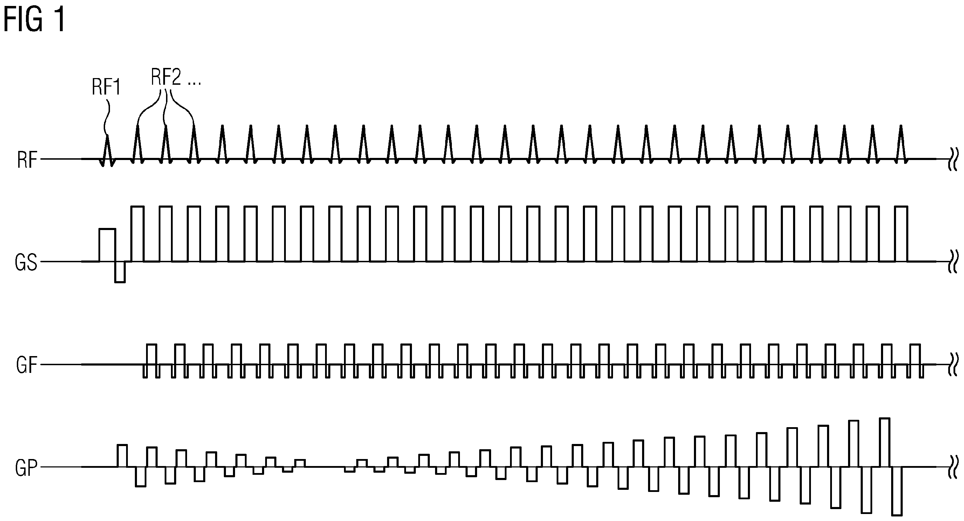

Figur 1 ist ein schematisches Sequenzdiagramm einer solchen HASTE-Sequenz in seinem zeitlichen Verlauf. Wie bereits erwähnt, werden nach einem Anregungspuls RF1 mehrere Refokussierungspulse RF2 geschaltet, um mehrere Spinecho-Signale zu erzeugen, die jeweils zwischen den Refokussierungspulsen RF2 entstehen (nicht dargestellt). Zur Beschränkung der Anregung auf Spins einer bestimmten Schicht kann hierbei gleichzeitig mit jedem Anregungspuls RF1 und Refokussierungspuls RF2 ein entsprechender Schichtselektionsgradient GS geschaltet werden. Zur weiteren Ortskodierung können Frequenzkodiergradienten GF und Phasenkodiergradienten GP geschaltet werden. Bei HASTE kann dabei insbesondere auf einige (in der Regel knapp die Hälfte der) Phasenkodiergradienten GP verzichtet werden, wie es in Figur 1 durch die asymmetrische Anordnung der Phasenkodiergradienten GP angedeutet ist. Die entsprechenden, "fehlenden" Spinechosignale können, wie gesagt, mit einem partial-fourier-Verfahren ergänzt werden. Die dargestellte asymmetrische Anordnung der Phasenkodiergradienten GP, bei der nach der Messung der Echosignale im k-Raumzentrum (GP = 0) eine größere Anzahl von Echosignalen gemessen wird, als vor der Messung des k-Raumzentrums, ist vorteilhaft, um die Echozeiten möglichst kurz zu halten, zu denen der Bereich des k-Raumzentrums (niedrige Amplituden der Phasenkodiergradienten GP) gemessen werden. Auf diese Weise sind die Echosignale des Bereichs des k-Raumzentrums weniger stark gemäß dem T2-Zerfall geschwächt als bei einer anderen Anordnung, die den Bereich des k-Raumzentrums erst zu späteren Echozeiten misst.

Neben den genannten Partial-Fourier-Verfahren sind weiterhin sogenannte parallele Akquisitionstechniken (z.B. GRAPPA ("GeneRalized Autocalibrating Partially Parallel Acquisition") und SENSE ("SENSitivity Encoding") bekannt, mit deren Hilfe sich zur Aufnahme der gewünschten Daten benötigte Akquisitionszeiten verkürzen lassen. Wie bei partial-fourier-Verfahren werden hierbei nur Teile der eigentlich gemäß der Nyquist-Bedingung als k-Raumdaten aufzunehmenden Echosignale gemessen. Im Gegensatz zu den partial-fourier-Verfahren sind jedoch in der Regel die nicht-gemessenen Teile bei parallelen Akquisitionstechniken gleichmäßiger über den gemäß Nyquist zu messenden k-Raum verteilt, sodass z.B. nur jede zweite k-Raumzeile gemessen wird. Darüber hinaus werden die "fehlenden" k-Raumdaten bei parallelen Akquisitionstechniken mit Hilfe von Spulensensitivitätsdaten rekonstruiert. Diese Spulensensitivitätsdaten der bei der Aufnahme der Messdaten verwendeten Empfangsspulen werden aus Referenzdaten ermittelt, die zumindest einen Bereich des zu messenden k-Raums, meist den zentralen Bereich, vollständig gemäß der Nyquist-Bedingung abtasten.

Der Wunsch nach immer schnelleren MR-Aufnahmen im klinischen Umfeld führt momentan zu einer Renaissance von Verfahren, bei denen mehrere Bilder simultan aufgenommen werden. Allgemein lassen sich diese Verfahren dadurch charakterisieren, dass zumindest während eines Teils der Messung gezielt Transversalmagnetisierung von zumindest zwei Schichten gleichzeitig für den Bildgebungsprozess genutzt wird ("Multi-Schicht-Bildgebung", "Schicht-Multiplexing"). Im Gegensatz dazu wird bei der etablierten "Mehrschicht-Bildgebung" das Signal von zumindest zwei Schichten alternierend, d.h. vollständig unabhängig voneinander mit entsprechend längerer Messzeit aufgenommen.

Bekannte Verfahren hierzu sind beispielsweise die sogenannte Hadamard-Kodierung, Verfahren mit simultaner Echo-Refokussierung, Verfahren mit Breitband-Datenaufnahme oder auch Verfahren, die eine parallele Bildgebung in Schicht-Richtung einsetzten. Zu den letztgenannten Verfahren gehören beispielsweise auch die CAIPIRINHA-Technik, wie sie von Breuer et al. in "Controlled Aliasing in Parallel Imaging Results in Higher Acceleration (CAIPIRINHA) for Multi-Slice Imaging", Magnetic Resonance in Medicine 53, 2005, S. 684-691 beschrieben ist, und die blipped CAIPIRINHA-Technik, wie sie von Setsompop et al. in "Blipped-Controlled Aliasing in Parallel Imaging for Simultaneous Multislice Echo Planar Imaging With Reduced g-Factor Penalty", Magnetic Resonance in Medicine 67, 2012, S. 1210-1224, beschrieben wird.

Insbesondere bei den letztgenannten Schicht-Multiplexing-Verfahren wird ein sogenannter Multi-Band-RF-Puls verwendet, um zwei oder mehr Schichten gleichzeitig (simultan) anzuregen oder anderweitig zu manipulieren, z.B. zu refokussieren oder zu sättigen. Ein solcher Multi-Band-RF-Puls ist dabei z.B. ein Multiplex von individuellen RF-Pulsen, die zur Manipulation der einzelnen gleichzeitig zu manipulierenden Schichten verwendet werden würden. Durch das Multiplexing erhält man z.B. einen grundbandmodulierten Multi-Band-RF-Puls aus einer Addition der Pulsformen der individuellen RF-Pulse. Die Ortskodierung der aufgenommenen Signale wird dabei im Wesentlichen durch eine gängige Gradientenschaltung in zwei Richtungen (zweidimensionale Gradientenkodierung) erreicht. Der Multi-Band-RF-Puls ist in seiner Wirkung räumlich selektiv auf die gewünschten Schichten beschränkt.

Die entstehenden Signale werden aus allen angeregten Schichten kollabiert in einem Datensatz mittels mehreren Empfangsantennen aufgenommen und dann mit Hilfe von parallelen Akquisitionstechniken nach den einzelnen Schichten getrennt.

Wie bereits weiter oben erwähnt, kann durch parallele Akquisitionstechniken bereits generell die zur Aufnahme der gewünschten Daten benötigte Akquisitionszeit durch eine gemäß Nyquist nicht vollständige, d.h. eine Unterabtastung des k-Raums verkürzen lassen.

Bei Schicht-Multiplexing-Verfahren werden parallele Akquisitionstechniken verwendet, um die gleichzeitig für verschiedene Schichten aufgenommenen Messdaten wieder zu trennen. Dabei müssen Referenzdaten für alle betroffenen Schichten aufgenommen werden. Dies geschieht in der Regel im Rahmen einer zusätzlich durchzuführenden Referenzmessung, die Referenzdaten einzeln für jede gewünschte Schicht misst.

Um die resultierenden Signale der verschiedenen Schichten trennen zu können, wird beispielsweise den individuellen RF-Pulsen vor dem Multiplexing, z.B. durch Addieren einer Phase, die linear (z.B. mit der k-Raumkoordinate in die Phasenkodierrichtung (k_{y})) steigt, je eine unterschiedliche Phase aufgeprägt. Damit kann jeder Schicht ein unterschiedlicher Phasenanstieg aufgeprägt werden, wodurch die Schichten im Bildraum gegeneinander verschoben werden. Diese Verschiebung wird durch den sogenannten Bildbereich-Verschiebungsfaktor ("FOV (field of view) shift factor") kontrolliert. Wie ein optimaler FOV shift factor bestimmt werden kann, wird beispielsweise in der nachveröffentlichten DE102016218955 beschrieben.

In den in den genannten Artikel von Breuer et al. und Setsompop et al. beschriebenen CAIPIRINHA-Verfahren werden durch Schalten von zusätzlichen Gradientenblips oder durch zusätzliches Modulieren der Phasen der RF-Pulse der Multi-Band-RF-Pulse zwischen den gleichzeitig angeregten Schichten wechselnde weitere Phasenverschiebungen aufgeprägt, die Verschiebungen im Bildraum erzeugen. Diese zusätzlichen Verschiebungen im Bildraum verbessern die Qualität der Trennung der Signale der Schichten, insbesondere, wenn die Spulensensitivitäten derartig geringe Unterschiede in den Sensitivitätsprofilen der einzelnen verwendeten Spulen aufweisen, dass diese nicht für eine zuverlässige Trennung der Schichten ausreichen. Somit werden Artefakte in den letztendlich aus den gemessenen Messdaten rekonstruierten Bilddaten verringert.

Die Referenzdaten, aus denen die für die Trennung der Schichten und/oder Ergänzung der fehlenden Messpunkte benötigten Sensitivitätsdaten gewonnen werden, müssen bisher für jede Schicht-Multiplexing-Messung zusätzlich gemessen werden.

Werden derartige Referenzdaten mit einem anderen Sequenztyp aufgenommen als die zu vervollständigenden Messdaten der eigentlichen Messung, kann das aufgrund von möglicherweise unterschiedlichen Kontrasten und/oder unterschiedlichen Empfindlichkeiten (Sensitivitäten) auf verschiedene Störungen zu Artefakten bei der Rekonstruktion der Messdaten führen. Auch der Zeitpunkt zu dem die Referenzdaten im Vergleich zu den eigentlichen Messdaten aufgenommen werden kann, z.B. durch (ungewollte) Bewegungen des Untersuchungsobjektes, zu Artefakten bei der Rekonstruktion führen. Dies ist auch ein Grund, warum Schicht-Multiplexing-Verfahren zwar bereits mit einer Vielzahl verschiedener Sequenztypen, unter anderem EPI und TSE, kombiniert werden, eine Kombination mit Single-Shot-TSE-Techniken aber bisher nicht zu befriedenden Ergebnissen führte.

Weiterhin können Abweichungen in den bei der Aufnahme der Referenzdaten verwendeten Messparametern von den bei der Multi-Band-Aufnahme verwendeten Messparametern, insbesondere Messparameter bezüglich der Eigenschaften der RF-Anregungspulse und/oder bezüglich des Auslesevorgangs, wie z.B. die Auslesebandbreite, die Qualität der Trennung der Schichten beeinflussen und zu ungewünschten Artefakten führen. Unter diesem Gesichtspunkt sind in der nachveröffentlichten US-Patentanmeldung 15/262,233 bereits verschiedene Verfahren beschrieben, auf welche Weise derartige Referenzdaten neben den Multi-Band-Messdaten gewonnen werden können.

In der nachveröffentlichten DE102016207641 ist bereits ein Verfahren beschrieben, wie Referenzdaten für Einzelschichtaufnahmen im Anschluss an die Aufnahme von Messdaten aufgenommen werden, um z.B. eine Empfindlichkeit gegenüber physiologischen Bewegungen zu reduzieren. Die Referenzdaten werden hierbei jedoch jeweils nur für die eine gemessene Schicht gemessen.

Der Erfindung liegt die Aufgabe zugrunde, die Vorteile von Single-Shot-Verfahren, insbesondere HASTE, mit den Vorteilen von Schicht-Multiplexing ohne Qualitätsverlust kombinierbar zu machen.

Die Aufgabe wird gelöst durch ein Verfahren zur Aufnahme von Magnetresonanzdaten gemäß Anspruch 1, eine Magnetresonanzanlage gemäß Anspruch 11, ein Computerprogramm gemäß Anspruch 12, sowie einen elektronisch lesbarer Datenträger gemäß Anspruch 13.

Ein erfindungsgemäßes Verfahren zum Erstellen von Magnetresonanzdaten von mindestens zwei sich nicht überschneidenden Schichten eines Untersuchungsobjekts mittels einer parallelen Akquisitionstechnik umfasst die Schritte:
- Einstrahlen eines Multi-Band-RF-Anregungspulses, der die Magnetisierung von mindestens zwei sich nicht überschneidenden Schichten simultan und selektiv manipuliert, in ein Zielvolumen eines Untersuchungsobjekts,
- Aufnehmen von durch den Multi-Band-RF-Anregungspuls erzeugten Echosignalen aus den mindestens zwei Schichten als Schichten-Messdaten in einem Schichten-Messdatensatz, wobei der Schichten-Messdatensatz Messdaten aller simultan manipulierten Schichten umfasst,
- Aufnehmen von Referenzdaten der mindestens zwei Schichten derart, dass in einem zentralen Bereich des durch die Schichten-Messdaten und die Referenzdaten befüllten k-Raums insgesamt ein vollständiger Datensatz vorliegt, wobei die Aufnahme der Referenzdaten für einen Schichten-Messdatensatz derart erfolgt, dass zwischen der Aufnahme der Schichten-Messdaten des Schichten-Messdatensatzes und der Aufnahme seiner zugehörigen Referenzdaten keine Messdaten von Schichten aufgenommen werden, welche nicht in den mindestens zwei Schichten des Schichten-Messdatensatzes umfasst sind,

- Erstellen von Kalibrierungsdaten aus dem vollständigen Datensatz,
- Trennen des Schichten-Messdatensatz in Einzelschicht-Messdatensätze der simultan manipulierten Schichten unter Verwendung der Kalibrierungsdaten,
- Rekonstruieren von Bilddaten aus den Einzelschicht-Messdatensätzen.

Durch die Aufnahme der Referenzdaten derart, dass zwischen der Aufnahme der Schichten-Messdaten des Schichten-Messdatensatzes und seiner zugehörigen Referenzdaten keine Schichten-Messdaten eines anderen Schichten-Messdatensatzes aufgenommen werden, erreicht das Verfahren eine hohe Robustheit gegenüber Bewegungen des Untersuchungsobjekts. Durch die zeitlich nahe beieinander liegende Aufnahme der Referenzdaten einerseits und der zugehörigen Schichten-Messdaten andererseits werden die Referenzdaten inhärent in einem gleichen Bewegungszustand des Untersuchungsobjekts aufgenommen wie die Schichten-Messdaten, wodurch Artefakte, wie sie bei unterschiedlichen Bewegungszuständen für Referenzdaten und Schichten-Messdaten entstehen, vermieden werden.

Dies ist insbesondere in Verbindung mit Single-Shot-TSE-Verfahren vorteilhaft, welche ansonsten besonders anfällig gegenüber bewegungsinduzierten Artefakten sind. Damit können die Vorteile von Schicht-Multiplexing-Verfahren, welche eine Aufnahme von Referenzdaten erfordern, mit den Vorteilen von Single-Shot-Verfahren kombiniert werden.

Durch die mit einem Schicht-Multiplexing-Verfahren mögliche Beschleunigung der Aufnahme der Messdaten kann somit bei Single-Shot-Verfahren z.B. eine Verkürzung einer insgesamt für die vollständige Datenaufnahme benötigten Atemanhaltezeit (beispielsweise bei abdominellen HASTE-Untersuchungen) sowohl für Messungen bei welchen der Patient nur einmal seinen Atem anhalten soll, als auch für Messungen bei welchen ein Patient mehrmals, für jeweils verschiedene Messdurchgänge, seinen Atem anhalten soll, erreicht werden.

Selbst wenn es nicht nötig oder gewünscht ist, die insgesamte Atemanhaltezeit zu verkürzen, kann mit dem hierin beschriebenen Verfahren erreicht werden, dass in derselben Atemanhaltezeit Messdaten aus mehr Schichten aufgenommen werden können und somit eine höhere Schichtabdeckung ermöglicht wird.

Insbesondere für Patienten mit reduzierter Atemanhaltefähigkeit bringt das hierin beschriebene Verfahren somit Vorteile mit sich, da durch das Verfahren bestimmte Aufnahmen für solche Patienten überhaupt erst ermöglicht werden, und auch, weil in jedem Fall der Komfort für den Patienten durch die Reduzierung der Aufnahmezeiten und/oder Atemanhaltezeiten deutlich erhöht wird.

Mit dem hierin beschriebenen Verfahren kann somit, insbesondere bei MR-Aufnahmen, bei welchen verlangt ist, dass ein Patient für die Dauer einer Datenaufnahme seinen Atem anhält, sichergestellt werden, dass keine großen Unterschiede in den Messbedingungen, insbesondere bezüglich einer Atemanhalteposition des Patienten, bei der Aufnahme der (Schichten- )Messdaten einerseits und bei der Aufnahme von Referenzdaten, die für die spätere Rekonstruktion der Bilddaten benötigt werden, andererseits auftreten. Damit werden unerwünschte Artefakte effizient vermieden.

Eine erfindungsgemäße Magnetresonanzanlage umfasst eine Magneteinheit, eine Gradienteneinheit, eine Hochfrequenzeinheit und eine zur Durchführung eines erfindungsgemäßen Verfahrens ausgebildete Steuereinrichtung mit einer Hochfrequenz-Sende-/Empfangs-Steuerung mit einer Multi-Band-RF-Pulseinheit.

Ein erfindungsgemäßes Computerprogramm implementiert ein erfindungsgemäßes Verfahren auf einer Steuereinrichtung, wenn es auf der Steuereinrichtung ausgeführt wird.

Das Computerprogramm kann hierbei auch in Form eines Computerprogrammprodukts vorliegen, welches direkt in einen Speicher einer Steuereinrichtung ladbar ist, mit Programmcode-Mitteln, um ein erfindungsgemäßes Verfahren auszuführen, wenn das Computerprogrammprodukt in der Recheneinheit des Rechensystems ausgeführt wird.

Ein erfindungsgemäßer elektronisch lesbarer Datenträger umfasst darauf gespeicherte elektronisch lesbare Steuerinformationen, welche zumindest ein erfindungsgemäßes Computerprogramm umfassen und derart ausgestaltet sind, dass sie bei Verwendung des Datenträgers in einer Steuereinrichtung einer Magnetresonanzanlage ein erfindungsgemäßes Verfahren durchführen.

Die in Bezug auf das Verfahren angegebenen Vorteile und Ausführungen gelten analog auch für die Magnetresonanzanlage, das Computerprogrammprodukt und den elektronisch lesbaren Datenträger.

Weitere Vorteile und Einzelheiten der vorliegenden Erfindung ergeben sich aus den im Folgenden beschriebenen Ausführungsbeispielen sowie anhand der Zeichnungen. Die aufgeführten Beispiele stellen keine Beschränkung der Erfindung dar. Es zeigen:
- Fig. 1: eine schematische Darstellung einer HASTE-Sequenz,
- Fig. 2-5: schematische Darstellungen verschiedener Abtastschemata des k-Raums für parallele Akquisitionstechniken,
- Fig. 6: ein schematisches Ablaufdiagramm eines erfindungsgemäßen Verfahrens,
- Fig. 7: ein Beispiel eines erfindungsgemäßen Abtastschemas des k-Raums,
- Fig. 8: eine schematische Darstellung eines mit einem Abtastschema gemäß Figur 7 erzeugten Signals,
- Fig. 9: ein weiteres Beispiel eines erfindungsgemäßen Abtastschemas des k-Raums,
- Fig. 10: eine schematische Darstellung eines mit einem Abtastschema gemäß Figur 9 erzeugten Signals,
- Fig. 11: ein weiteres Beispiel eines erfindungsgemäßen Abtastschemas des k-Raums,
- Fig. 12: eine schematische Darstellung eines mit einem Abtastschema gemäß Figur 11 erzeugten Signals,
- Fig. 13: eine schematische Darstellung einer erfindungsgemäßen Magnetresonanzanlage.

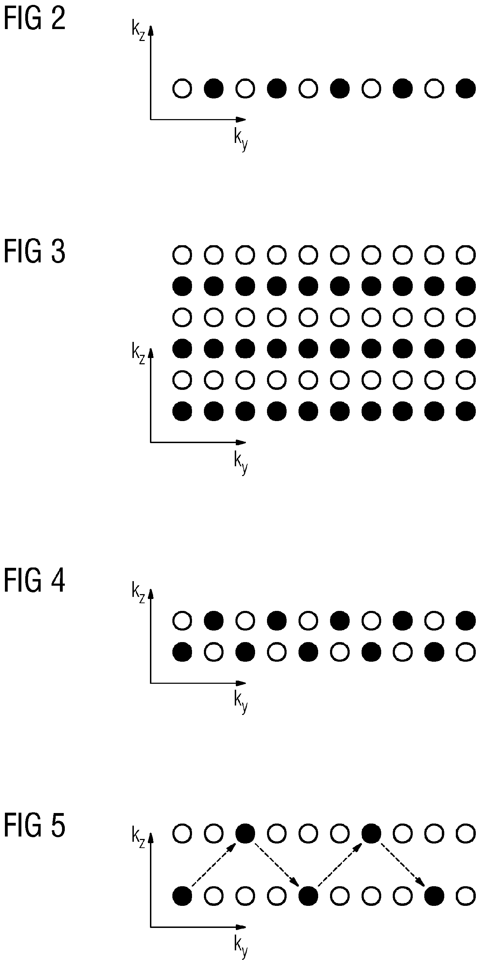

In den Figuren 2 bis 5 sind beispielhaft verschiedenen Abtastschemata des k-Raums für GRAPPA-artige parallele Bildgebungstechniken, jeweils mit einem kartesischen Abtastschema, gegenübergestellt. Die kₓ-Richtung liegt hierbei senkrecht zur Blattebene und das Abtastschema ist in kₓ-Richtung immer gleich, sofern eine Abtastung in kₓ-Richtung erfolgt. Die gefüllten Kreise repräsentieren gemessene k-Raumpunkte, die leeren Kreise ausgelassene k-Raumpunkte. Die Anzahl der tatsächlich zu messenden k-Raumpunkte kann deutlich höher sein, als in den Abtastschemata zeigenden Figuren dargestellt. Die gezeigten Abtastschemata veranschaulichen jeweils nur die zugrunde liegende Abtastschematik.

Figur 2 zeigt eine herkömmliche Einzelschicht-GRAPPA-Abtastung, in der jede zweite k-Raumlinie in einer Raumrichtung (hier: k_{y}-Richtung) ausgelassen wird, und somit nur die Hälfte der k-Raumpunkte gemessen werden, was einem Beschleunigungsfaktor von 2 entspricht.

Figur 3 zeigt eine herkömmliche dreidimensionale (3D) GRAPPA-Abtastung, die k-Raumlinien in der ky-kx-Ebene abtastet, und bei der jede zweite k-Raumlinie in einer Raumrichtung (hier: kz-Richtung) ausgelassen wird, und somit nur die Hälfte der k-Raumpunkte gemessen werden, womit sich auch hier ein Beschleunigungsfaktor 2 ergibt.

Die Wirkung der zusätzlichen Phasenverschiebungen auf das Abtastschema einer zweidimensionalen (2D) Schicht-Multiplexing-Messung ist in Figur 4 dargestellt. Durch die zusätzlichen Phasen, wie sie z.B. in Schicht-Multiplexing CAIPIRNHA-Verfahren aufgeprägt werden, werden die mit der zusätzlichen Phase beaufschlagten Messpunkte im k-Raum verschoben. Im in Figur 4 dargestellten Beispiel wird jeder zweiter k-Raumpunkt in k_{y}-Richtung mit einer Phase beaufschlagt, die zu einer Verschiebung in k_{z}-Richtung führt. Wie groß diese Verschiebung in k_{z}-Richtung ausfällt, hängt von der aufgeprägten Phase ab. Dies ist beispielsweise auch in dem Artikel von Zahneisen et al.: "Three-Dimensional Fourier Encoding of Simultaneously Excited Slices: Generalized Acquisition and Reconstruction Framework", Magn. Reson. Med. 71, S. 2071-2081 (2014), beschrieben. Eine zweidimensionale (2D) Schicht-Multiplexing-Messung mit durch zusätzliche Gradienten in die dritte (senkrecht auf die 2D-Ebene stehende) k-Raumrichtung aufgeprägten Verschiebungen kann somit analog zu einer 3D CAIPIRIHNA-Messung betrachtet werden. Um einen auf diese Weise aufgenommenen Datensatz zu rekonstruieren, wird ein vollständiger Referenzdatensatz benötigt. Mit Hilfe von parallelen Akquisitionstechniken und auf Basis des Referenzdatensatzes kann sowohl eine Trennung der aufgenommenen Messdaten in die verschiedenen Schichten als auch eine Vervollständigung von unterabgetasteten Daten einer Schicht erfolgen.

In Figur 5 ist ein Abtastschema dargestellt, bei dem die in Figur 2 dargestellte Abtastung mit einem Schicht-Multiplexing wie es in Figur 4 gezeigt ist, kombiniert ist. Somit werden in k_{y}-Richtung bereits nur jeder zweite k-Raumpunkt gemessen (wie in Figur 2) und zusätzlich wird jeder zweite gemessene k-Raumpunkt mit einer zusätzlichen Phase beaufschlagt, wodurch eine Verschiebung (hier) in k_{z}-Richtung erfolgt.

Wird dieses Abtastschema wie ein 3D-Schema betrachtet, folgt, dass in jeder k_{y}-Zeile jeweils nur ein k-Raumdatenpunkt aufgenommen wird und die folgenden drei k-Raumdatenpunkte nicht aufgenommen werden. Somit müssen in jeder k_{y}-Zeile jeweils drei k-Raumpunkte zwischen zwei aufgenommenen k-Raumpunkten durch Rekonstruktion ergänzt werden, um einen vollständigen Datensatz zu erhalten.

Ein derartiges Abtastschema kann beispielsweise mit einem HASTE-Verfahren mit einem Multi-Band-RF-Anregungspuls, der zwei Schichten simultan anregt, gefolgt von einer Reihe von Multi-Band-RF-Refokussierungspulsen, die die Spins in den zwei Schichten refokussieren, und Aufnahme der so erzeugten Echosignale als Messdatensätze, in denen die Messdaten der Schichten überlagert vorliegen, erreicht werden.

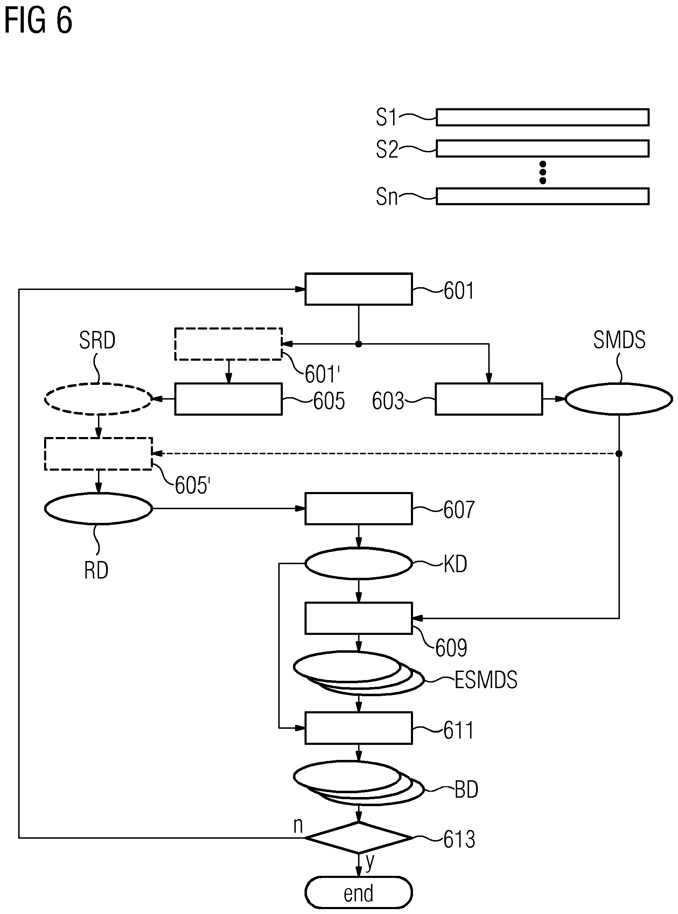

Figur 6 ist ein schematisches Ablaufdiagramm eines erfindungsgemäßen Verfahrens zum Erstellen von Magnetresonanzdaten von mindestens zwei sich nicht überschneidenden, insbesondere parallelen, Schichten eines Untersuchungs-objektes mittels einer parallelen Akquisitionstechnik.
Dabei wird ein Multi-Band-RF-Anregungspuls in ein Zielvolumen (FOV) eines Untersuchungsobjekts eingestrahlt (Block 601). Der Multi-Band-RF-Anregungspuls manipuliert die Magnetisierung von mindestens zwei sich nicht überschneidenden Schichten S1, S2, ... Sn simultan und selektiv, insbesondere regt er die Spins in den mindestens zwei sich nicht überschneidenden Schichten S1, S2, ... Sn simultan und selektiv an.

Die durch den Multi-Band-RF-Anregungspuls erzeugten Echosignale aus den mindestens zwei Schichten werden als Schichten-Messdaten aufgenommen (Block 603) und in einem Schichten-Messdatensatz SMDS gespeichert. Die Schichten-Messdaten entsprechen den überlagert aus den mindestens zwei Schichten aufgenommenen Messdaten. Der Schichten-Messdatensatz SMDS umfasst somit Messdaten aller simultan manipulierter Schichten. Hierbei können insbesondere alle Echosignale eines Schichten-Messdatensatzes SMDS nach einem einzigen Multi-Band-RF-Puls aufgenommen werden (Single-Shot). Die Aufnahme der Magnetresonanzdaten kann hierbei gemäß einer Turbo-Spinecho-Sequenz, insbesondere gemäß einer HASTE-Sequenz, erfolgen.

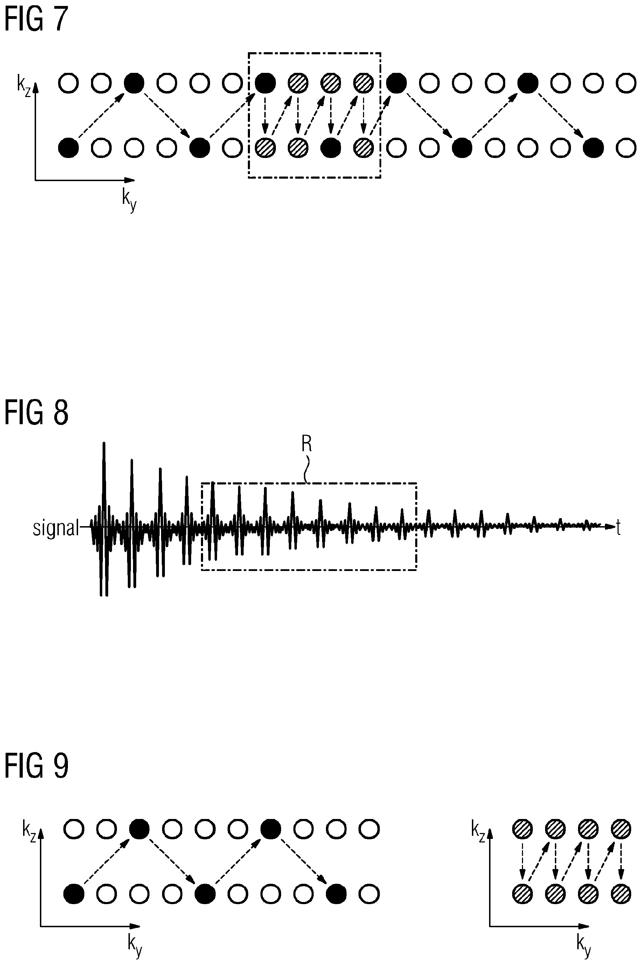

Zu den in Block 603 aufgenommenen Schichten-Messdaten gehörende Referenzdaten RD der mindestens zwei Schichten werden aufgenommen (Block 605) derart, dass in einem zentralen Bereich des durch die aufgenommenen Schichten-Messdaten des Schichten-Messdatensatzes SMDS und die aufgenommenen Referenzdaten befüllten k-Raums insgesamt ein gemäß Nyquist vollständiger Datensatz vorliegt. Dabei erfolgt die Aufnahme der Referenzdaten RD für einen Schichten-Messdatensatz SMDS derart, dass zwischen der Aufnahme 603 der Schichten-Messdaten des Schichten-Messdatensatzes SMDS und der Aufnahme 605 seiner zugehörigen Referenzdaten RD keine Schichten-Messdaten eines anderen Schichten-Messdatensatzes aufgenommen werden. Mit anderen Worten werden zwischen der Aufnahme 603 der Schichten-Messdaten des Schichten-Messdatensatzes SMDS und der Aufnahme 605 seiner zugehörigen Referenzdaten RD keine Messdaten von Schichten aufgenommen, welche nicht in den mindestens zwei Schichten des Schichten-Messdatensatzes umfasst sind. Die Aufnahme 605 der Referenzdaten RD erfolgt somit insbesondere nach dem Einstrahlen (601) des Multi-Band-RF-Anregungspulses und spätestens unmittelbar anschließend an die Aufnahme 603 der Schichten-Messdaten.
Das Aufnehmen 605 der Referenzdaten RD kann nach Einstrahlen eines weiteren RF-Pulses erfolgen. Beispielsweise kann das Aufnehmen 605 der Referenzdaten RD nach Einstrahlen eines zweiten Multi-Band-RF-Pulses zur simultanen und selektiven Manipulation der mindestens zwei Schichten erfolgen, wobei die Referenzdaten RD z.B. als Schichten-Referenzdaten SRD, die die Referenzdaten der mindestens zwei Schichten überlagert enthalten, gespeichert werden können. Die erhaltenen Schichten-Referenzdaten SRD können in Referenzdaten RD getrennt werden, die jeweils nur Referenzdaten einer der mindestens zwei Schichten umfassen (Block 605'). Zur Trennung der Schichten-Referenzdaten SRD in (Einzelschicht-)Referenzdaten RD kann beispielsweise eine Fourier-Transformation in einer k-Raumrichtung, in der durch zusätzliche Gradienten eine Verschiebung erzeugt wurde, wie es in dem genannten Artikel von Zahneisen et al. beschrieben ist, eingesetzt werden. Hierzu wird weiter unten in Bezug auf die Figuren 7 bis 10 weiter ausgeführt.

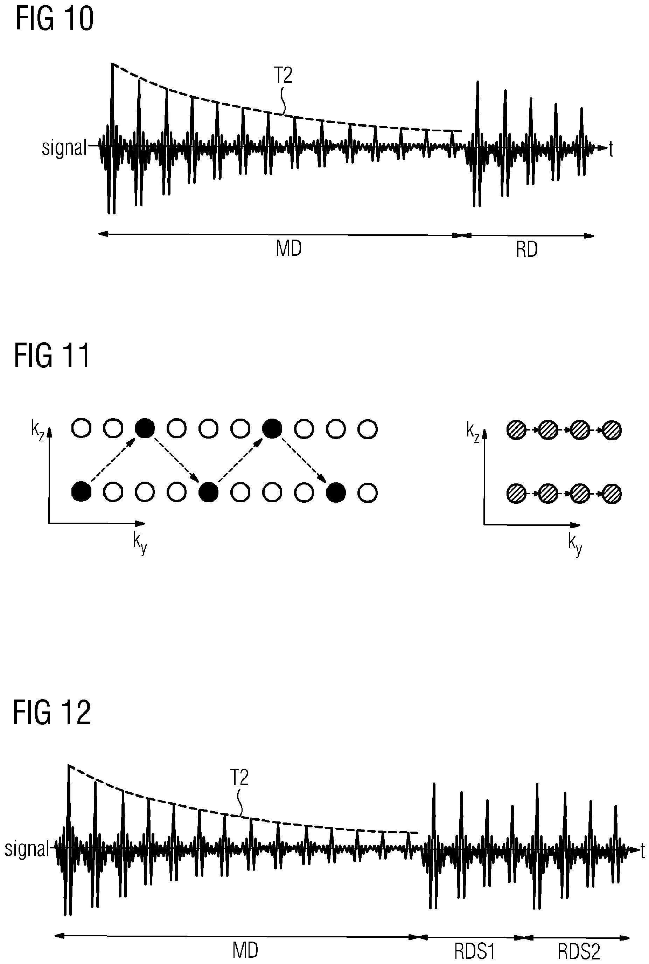

Es ist auch möglich das Aufnehmen 605 der Referenzdaten RD nach Einstrahlen von Einzelschicht-RF-Anregungspulsen für jede der mindestens zwei Schichten durchzuführen, wobei die Referenzdaten RD direkt als jeweilige Referenzdaten RD für jede der mindestens zwei Schichten aufgenommen und gespeichert werden. Hierzu wird weiter unten in Bezug auf die Figuren 11 und 12 weiter ausgeführt.

Aus dem erhaltenen vollständigen Datensatz werden Kalibrierungsdaten KD erstellt (Block 607). Aus den Kalibrierungsdaten KD können alle jeweils benötigten Kalibrierungsdatensätze, z.B. für die Trennung der Schichten-Messdatensätze SMDS in Einzelschicht-Messdatensätze ESMDS, wie auch für die Ergänzung von nicht-gemessenen k-Raumdaten innerhalb einer Schicht, entsprechend der jeweils vorliegenden Unterabtastung gewonnen werden. Die Kalibrierungsdaten KD können hierbei auch als dreidimensionale (3D) Kalibrierungsdaten KD gewonnen werden, mit welchen fehlende Messpunkte im wie in dem Artikel von Zahneisen et al. als dreidimensional betrachteten k-Raum gleichzeitig ergänzt werden.

Der aufgenommene Schichten-Messdatensatz SMDS wird unter Verwendung der Kalibrierungsdaten KD in Einzelschicht-Messdatensätze ESMDS der simultan manipulierten Schichten S1, S2, ... Sn getrennt (Block 609). Hierbei kann eine parallele Akquisitionstechnik verwendet werden.

Aus den Einzelschicht-Messdatensätzen ESMDS können nun Bilddaten der mindestens zwei Schichten rekonstruiert werden (Block 611).

Sind die nach Block 609 erhaltenen Einzelschicht-Messdatensätze ESMDS gemäß Nyquist unterabgetastet, kann das Rekonstruieren der Bilddaten BD eine Vervollständigung der Einzelschicht-Messdatensätze ESMDS unter Verwendung der Kalibrierungsdaten KD umfassen. Insbesondere kann aus den Kalibrierungsdaten KD ein der Unterabtastung der Einzelschicht-Messdatensätze entsprechender Kalibrierungsdatensatz erstellt werden, mit welchem die Einzelschicht-Messdatensätze ESMDS ergänzt werden können bevor Bilddaten rekonstruiert werden.

Sind somit Bilddaten aller gewünschten Schichten S1, S2, ... Sn erstellt ("y", Abfrage 613), endet das Verfahren.

Wurden noch nicht alle gewünschten Schichten gemessen ("n", Abfrage 613), kann das Verfahren erneut bei Block 601 beginnend wiederholt werden, um die fehlenden Daten aufzunehmen. Insbesondere, wenn ein Single-Shot-Verfahren verwendet wird, bei dem alle aufzunehmenden Messdaten der manipulierten Schichten nach nur einem Multi-Band-RF-Anregungspuls aufgenommen werden, wird bei einer Wiederholung des Verfahrens gemäß der Schritte 601 bis 613 ein Multi-Band-RF-Anregungspuls verwendet, der eine Gruppe von mindestens zwei Schichten manipuliert, die bisher noch nicht von einem vorhergehenden Multi-Band-RF-Anregungspuls manipuliert wurden, und deren Messdaten somit noch nicht aufgenommen wurden, und von denen somit bisher auch noch keine Bilddaten rekonstruiert wurden.

Figur 7 ist ein Beispiel eines Abtastschemas des k-Raums wie es in dem erfindungsgemäßen Verfahren verwendet werden kann. Bei dem dargestellten Abtastschema werden k-Raumpunkte als Schichten-Messdaten analog zu einem beschleunigten Abtastschema gemäß Figur 5 gemessen (gemessene Punkte sind durch schwarz gefüllte Kreise dargestellt). Im Unterschied zu Figur 5 wird jedoch ein zentraler Bereich des k-Raums (gekennzeichnet durch eine gestrichelte Einrahmung) vollständig abgetastet, wobei zumindest die als schraffierte Kreise dargestellten k-Raumpunkte als Referenzdaten zusätzlich aufgenommen werden. Dies kann erreicht werden, indem in dem zentralen k-Raumbereich jede k_{y}-Position zweimal gemessen wird, wobei z.B. bei jeder zweiten Messung einer k_{y}-Position ein zusätzlicher Gradient zur Verschiebung in k_{z}-Richtung angewandt wird. Die Aufnahme der Magnetresonanzdaten erfolgt hierbei somit insbesondere gemäß einem CAIPIRINHA-Verfahren.

Die dargestellte Größe des zentralen k-Raumbereichs im Vergleich zu dem dargestellten abgetasteten k-Raumbereich ist nicht repräsentativ, sondern dient lediglich der besseren Veranschaulichung. Die tatsächliche Größe des zentralen k-Raumbereichs, in welchem die Referenzdaten aufgenommen werden, kann auf bekannte Art und Weise festgelegt werden.

Die Referenzdaten werden in dem in Figur 7 gezeigten Beispiel somit verschachtelt mit den Schichten-Messdaten nach einem einzigen gemeinsamen Multi-Band-RF-Anregungspuls aufgenommen und liegen zunächst als Schichten-Referenzdaten, in denen die Referenzdaten der einzelnen gemessenen Schichten überlagert sind, vor. Eine Trennung der Schichten-Referenzdaten in Referenzdaten für jeweils eine einzelne der aufgenommenen Schichten kann z.B. leicht über eine Fourier-Transformation in k_{z}-Richtung erfolgen. Die erhaltenen Referenzdaten können erfindungsgemäß sowohl für die Trennung der aufgenommenen Schichten-Messdaten in Einzelschicht-Messdaten als auch für die Ergänzung von unterabgetasteten Einzelschicht-Messdaten verwendet werden.

In Figur 8 ist schematisch ein beispielhaftes Signal in seinem zeitlichen Verlauf dargestellt wie es mit einer einem Abtastschema gemäß Figur 7 folgenden Sequenz unter Verwendung einer HASTE-Sequenz erzeugt werden kann. In dem mit R gekennzeichneten Zeitfenster werden die Referenzdaten aufgenommen. Die Stärke der Echosignale fällt mit der Zerfallsrate der transversalen Magnetisierung T2 ab.

Mit einer derartigen verschachtelten Aufnahme der Schichten-Messdaten und der Referenzdaten unter Verwendung eines Schicht-Multiplexing-Verfahrens werden die Referenzdaten und die Messdaten inhärent unter gleichartigen Bedingungen, insbesondere in einer selben physiologischen Bewegungsphase und zu gleichartigen Bildgebungsbedingungen (wie beispielsweise bei gleichen Schichtprofilen, gleichen Intensitäts- und/oder Anregungswinkelevolutionen, aufgenommen. Dadurch passen Referenzdaten und Messdaten optimal zueinander und Artefakte können vermieden werden.

Weiterhin können die aufgenommenen (Schichten-)Referenzdaten zu den Schichten-Messdaten hinzugenommen werden, bevor diese in Einzelschicht-Messdaten getrennt werden. Durch diese zusätzlichen Messdaten kann das Signal-zu-Rausch-Verhältnis (SNR) verbessert werden.

Figur 9 ist ein weiteres Beispiel eines Abtastschemas des k-Raums wie es in dem erfindungsgemäßen Verfahren verwendet werden kann. In diesem Beispiel werden die Schichten-Messdaten gemäß dem links dargestellten Abtastschema mit Schicht-Multiplexing-Beschleunigung (simultane Manipulation zweier Schichten) und mit einer parallelen Akquisitionsbeschleunigung mit Faktor 2 in k_{y}-Richtung analog zu Figur 5 aufgenommen.

Zeitlich direkt im Anschluss an die Aufnahme der Schichten-Messdaten (Zeitraum MD) werden Referenzdaten aufgenommen (Zeitraum RD). Dazu wird nach der Aufnahme der Schichten-Messdaten ein weiterer, zweiter Multi-Band-RF-Anregungspuls eingestrahlt, der wieder dieselben Schichten manipuliert, wie der Multi-Band-RF-Anregungspuls, der die als Schichten-Messdaten aufgenommenen Signale erzeugt hat.

Die Referenzdaten können dabei gemäß dem rechts dargestellten Abtastschema aufgenommen werden, das den zentralen k-Raumbereich zeigt. Ein solches Abtastschema kann analog zu dem mit Bezug auf den zentralen Bereich des Abtastschemas gemäß Figur 7 beschriebenen Vorgehen erreicht werden. Dabei kann bei der Aufnahme der Referenzdaten insbesondere derselbe Sequenztyp verwendet werden wie bei der Aufnahme der Schichten-Messdaten. Beispielsweise wenn die Schichten-Messdaten mittels einem (Schicht-Multiplexing-)HASTE-Verfahren aufgenommen werden, können die Referenzdaten ebenfalls mit Hilfe eines (Schicht-Multiplexing-)HASTE-Verfahrens aufgenommen werden.

Die somit wiederum zunächst als Schichten-Referenzdaten vorliegenden Referenzdaten können ebenso wie mit Bezug auf das Beispiel gemäß der Figuren 7 und 8 in Referenzdaten der einzelnen Schichten mit Hilfe einer Fourier-Transformation getrennt werden. Die erhaltenen Referenzdaten können erfindungsgemäß sowohl für die Trennung der aufgenommenen Schichten-Messdaten in Einzelschicht-Messdaten als auch für die Ergänzung von unterabgetasteten Einzelschicht-Messdaten verwendet werden.

In Figur 10 ist schematisch ein beispielhaftes Signal in seinem zeitlichen Verlauf dargestellt wie es mit einer einem Abtastschema gemäß Figur 9 folgenden Sequenz unter Verwendung einer HASTE-Sequenz erzeugt werden kann. In dem Zeitfenster MD werden die Schichten-Messdaten aufgenommen, wobei die Stärke der Echosignale mit der Zerfallskonstante T2 abfällt. Im Anschluss an die Aufnahme der Schichten-Messdaten werden die Referenzdaten im Zeitfenster RD aufgenommen, die nach einem erneuten Multi-Band-RF-Anregungspuls zunächst wieder eine größere Signalstärke aufweisen, die dann wiederum mit T2 abfällt.

Mit einer derartigen Aufnahme der Referenzdaten weiterhin unter Verwendung eines Schicht-Multiplexing-Verfahrens als Schichten-Referenzdaten direkt im Anschluss an die Aufnahme der Schichten-Messdaten wird sichergestellt, dass die Referenzdaten zumindest in einer ähnlichen, da zeitlich eng benachbarten, physiologischen Bewegungsphase aufgenommen werden wie die Schichten-Messdaten. Auch die Bildgebungsbedingungen, insbesondere das Schichtprofil (da sämtliche Multi-Band-RF-Anregungs- und -Refokussierungspulse auf dieselben Schichten wirken), die bei der Aufnahme der Referenzdaten vorliegen, stimmen weitgehend mit denjenigen überein, die bei der Aufnahme der Schichten-Messdaten vorlagen. Die im Verlauf der Zeit stets gleichförmige Aufnahme der Schichten-Messdaten im Zeitraum MD, trägt zusätzlich zu einer Vermeidung z.B. von Blurring-Artefakten bei.

Figur 11 ist ein weiteres Beispiel eines Abtastschemas des k-Raums wie es in dem erfindungsgemäßen Verfahren verwendet werden kann.

In diesem Beispiel werden die Schichten-Messdaten wiederum gemäß dem links dargestellten Abtastschema mit Schicht-Multiplexing-Beschleunigung (simultane Manipulation zweier Schichten) und mit einer parallelen Akquisitionsbeschleunigung mit Faktor 2 in k_{y}-Richtung analog zu Figur 5 und anaklog zu der Aufnahme der Schichten-Messdaten gemäß Figur 9 aufgenommen.

Zeitlich direkt im Anschluss an die Aufnahme der Schichten-Messdaten (Zeitraum MD) werden Referenzdaten jeweils einzeln für die Schichten, deren Messdaten in den Schichten-Messdaten enthalten sind, aufgenommen (Zeiträume RDS1 und RDS2). Dazu wird nach der Aufnahme der Schichten-Messdaten ein Einzelschicht-RF-Anregungspuls eingestrahlt, der beispielsweise eine erste der durch den vorhergehenden Multi-Band-RF-Puls manipulierten Schichten anregt, und somit Echosignale erzeugt, die als Referenzdaten RDS1 dieser Schicht aufgenommen werden. Wiederum direkt im Anschluss an die Aufnahme dieser Referenzdaten der ersten Schicht wird ein weiterer Einzelschicht-RF-Anregungspuls eingestrahlt, der beispielsweise eine zweite der durch den vorhergehenden Multi-Band-RF-Puls manipulierten Schichten anregt, und somit Echosignale erzeugt, die als Referenzdaten RDS2 dieser Schicht aufgenommen werden. Sollten weitere Schichten in dem Schichten-Messdaten enthalten sein, können analog weitere Referenzdaten für diese Schichten aufgenommen werden.

Die Referenzdaten können dabei für jede Schichtposition z gemäß dem rechts dargestellten Abtastschema aufgenommen werden, das den zentralen k-Raumbereich zeigt. Bei der Aufnahme der Referenzdaten kann insbesondere derselbe Sequenztyp verwendet werden wie bei der Aufnahme der Schichten-Messdaten. Beispielsweise wenn die Schichten-Messdaten mittels einem (Schicht-Multiplexing-)HASTE-Verfahren aufgenommen werden, können die Referenzdaten ebenfalls mit Hilfe eines (Einzelschicht-)HASTE-Verfahrens aufgenommen werden. Die erhaltenen Referenzdaten können erfindungsgemäß sowohl für die Trennung der aufgenommenen Schichten-Messdaten in Einzelschicht-Messdaten als auch für die Ergänzung von unterabgetasteten Einzelschicht-Messdaten verwendet werden.

In Figur 12 ist schematisch ein beispielhaftes Signal in seinem zeitlichen Verlauf dargestellt wie es mit einer einem Abtastschema gemäß Figur 11 folgenden Sequenz unter Verwendung einer HASTE-Sequenz erzeugt werden kann. In dem Zeitfenster MD werden die Schichten-Messdaten aufgenommen, wobei die Stärke der Echosignale mit der Zerfallskonstante T2 abfällt. Im Anschluss an die Aufnahme der Schichten-Messdaten werden die Referenzdaten für die einzelnen Schichten jeweils in den Zeitfenster RDS1 und RDS2 aufgenommen. Die Signalstärke fällt auch hier jeweils nach den zugehörigen RF-Anregungspuls gemäß der Zerfallskonstante T2 ab.

Mit einer derartigen Aufnahme der Referenzdaten als (Einzelschicht-)Referenzdaten unter Verwendung von Einzelschicht-Verfahrens jedoch wiederum direkt im Anschluss an die Aufnahme der Schichten-Messdaten, wird sichergestellt, dass die Referenzdaten zumindest in einer ähnlichen, da zeitlich eng benachbarten, physiologischen Bewegungsphase aufgenommen werden wie die Schichten-Messdaten. Die im Verlauf der Zeit stets gleichförmige Aufnahme der Schichten-Messdaten im Zeitraum MD, trägt zusätzlich zu einer Vermeidung z.B. von Blurring-Artefakten bei.

Die dargestellten Beispiele zeigen der besseren Vergleichbarkeit halber jeweils Verfahren zur Aufnahme von Schichten-Messdaten aus zwei Schichten ("SMS-Beschleunigungsfaktor 2") in Kombination mit einem weiteren Beschleunigungsfaktor 2 durch Verwendung paralleler Akquisitionstechniken (,d.h. nur jeder zweite k-Raumpunkt in einer k-Raumrichtung (hier: ky-Richtung) wird aufgenommen). Das Verfahren ist jedoch analog auf andere SMS-Beschleunigungsfaktoren und andere Beschleunigungsfaktoren der parallelen Akquisitionstechniken anwendbar.

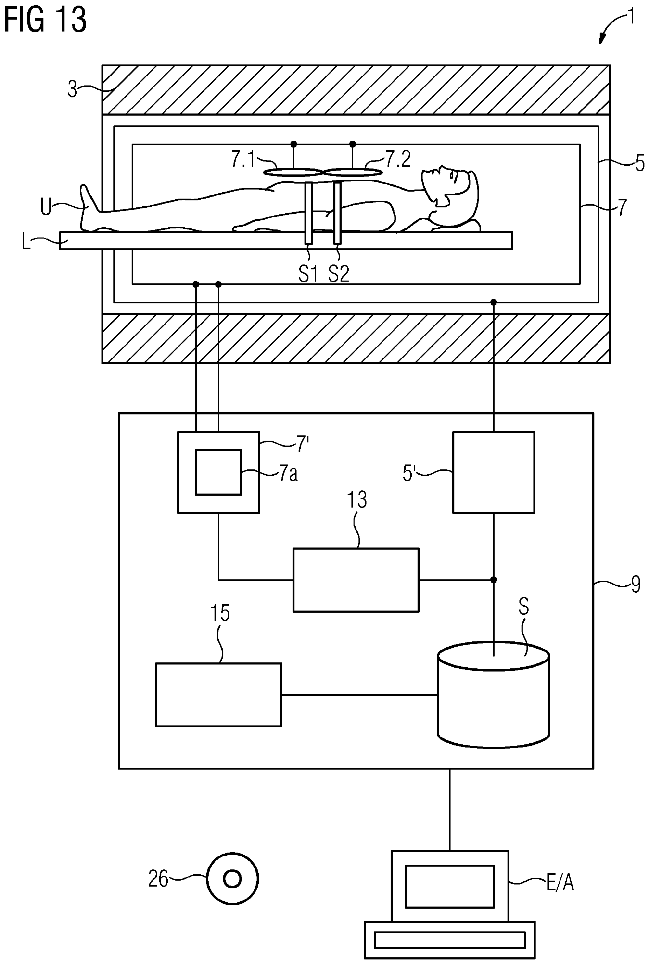

Figur 13 stellt schematisch eine erfindungsgemäße Magnetresonanzanlage 1 dar. Diese umfasst eine Magneteinheit 3 zur Erzeugung des Grundmagnetfeldes, eine Gradienteneinheit 5 zur Erzeugung der Gradientenfelder, eine Hochfrequenzeinheit 7 zur Einstrahlung und zum Empfang von Hochfrequenzsignalen und eine zur Durchführung eines erfindungsgemäßen Verfahrens ausgebildete Steuereinrichtung 9. In der Figur 13 sind diese Teileinheiten der Magnetresonanzanlage 1 nur grob schematisch dargestellt. Insbesondere besteht die Hochfrequenzeinheit 7 aus mehreren Untereinheiten, insbesondere aus mindestens zwei Spulen wie den schematisch gezeigten Spulen 7.1 und 7.2, die entweder nur zum Senden von Hochfrequenzsignalen oder nur zum Empfangen der ausgelösten Hochfrequenzsignale oder für beides ausgestaltet sein können.

Zur Untersuchung eines Untersuchungsobjektes U, beispielsweise eines Patienten oder auch eines Phantoms, kann dieses auf einer Liege L in die Magnetresonanzanlage 1 in deren Messvolumen eingebracht werden. Die Schichten S1 und S2 stellen exemplarisch zwei unterschiedlichen Schichten S1 und S2 des Untersuchungsobjekts dar, die bei einer Aufnahme von MR-Signalen gleichzeitig gemessen werden können.

Die Steuereinrichtung 9 dient der Steuerung der Magnetresonanzanlage und kann insbesondere die Gradienteneinheit 5 mittels einer Gradientensteuerung 5' und die Hochfrequenzeinheit 7 mittels einer Hochfrequenz-Sende-/Empfangs-Steuerung 7' steuern. Die Hochfrequenzeinheit 7 kann hierbei mehrere Kanäle umfassen, auf denen Signale gesendet oder empfangen werden können.

Die Hochfrequenzeinheit 7 ist zusammen mit ihrer Hochfrequenz-Sende-/Empfangs-Steuerung 7' für die Erzeugung und das Einstrahlen (Senden) eine Hochfrequenz-Wechselfeldes zur Manipulation der Spins in einem zu manipulierenden Bereich (insbesondere in verschiedene Schichten S1 und S2) des Untersuchungsobjekt U zuständig. Dabei sollte die Mittenfrequenz des auch als B1-Feld bezeichneten Hochfrequenz-Wechselfeldes nahe der Resonanzfrequenz der zu manipulierenden Spins liegen. Zur Erzeugung des B1-Feldes werden in der Hochfrequenzeinheit 7 mittels der Hochfrequenz-sende/empfangs-Steuerung 7' gesteuerte Ströme an den HF-Spulen angelegt.

Weiterhin umfasst die Steuereinrichtung 9 eine Phasenbestimmungseinheit 15 insbesondere zum Bestimmen von erfindungsgemäß zusätzlich aufzuprägenden Phasen.

Eine von der Steuereinrichtung 9 umfasste Recheneinheit 13 ist dazu ausgebildet alle für die nötigen Messungen und Bestimmungen nötigen Rechenoperationen auszuführen. Hierzu benötigte oder hierbei ermittelte Zwischenergebnisse und Ergebnisse können in einer Speichereinheit S der Steuereinrichtung 9 gespeichert werden. Die dargestellten Einheiten sind hierbei nicht unbedingt als physikalisch getrennte Einheiten zu verstehen, sondern stellen lediglich eine Untergliederung in Sinneinheiten dar, die aber auch z.B. in weniger oder auch in nur einer einzigen physikalischen Einheit realisiert sein können.

Über eine Ein-/Ausgabeeinrichtung E/A der Magnetresonanzanlage 1 können, z.B. durch einen Nutzer, Steuerbefehle an die Magnetresonanzanlage geleitet werden und/oder Ergebnisse der Steuereinrichtung 9 wie z.B. Bilddaten angezeigt werden.

Ein hierin beschriebenes Verfahren kann auch in Form eines Computerprogrammprodukts vorliegen, welches ein Programm umfasst und das beschriebene Verfahren auf einer Steuereinrichtung 9 implementiert, wenn es auf der Steuereinrichtung 9 ausgeführt wird. Ebenso kann ein elektronisch lesbarer Datenträger 26 mit darauf gespeicherten elektronisch lesbaren Steuerinformationen vorliegen, welche zumindest ein solches eben beschriebenes Computerprogrammprodukt umfassen und derart ausgestaltet sind, dass sie bei Verwendung des Datenträgers 26 in einer Steuereinrichtung 9 einer Magnetresonanzanlage 1 das beschriebene Verfahren durchführen.

## Patentansprüche

1. Verfahren zum Erstellen von Magnetresonanzdaten von mindestens zwei sich nicht überschneidenden Schichten eines Untersuchungsobjektes mittels einer parallelen Akquisitionstechnik umfassend die Schritte:
- Einstrahlen eines Multi-Band-RF-Anregungspulses, der die Magnetisierung von mindestens zwei sich nicht überschneidenden Schichten simultan und selektiv manipuliert, in ein Zielvolumen eines Untersuchungsobjekts,
- Aufnehmen von durch den Multi-Band-RF-Anregungspuls erzeugten Echosignalen aus den mindestens zwei Schichten als Schichten-Messdaten in einem Schichten-Messdatensatz, wobei der Schichten-Messdatensatz Messdaten aller simultan manipulierten Schichten umfasst,
- Aufnehmen von Referenzdaten der mindestens zwei Schichten derart, dass in einem zentralen Bereich des durch die Schichten-Messdaten und die Referenzdaten befüllten k-Raums insgesamt ein vollständiger Datensatz vorliegt, wobei die Aufnahme der Referenzdaten für einen Schichten-Messdatensatz derart erfolgt, dass zwischen der Aufnahme der Schichten-Messdaten des Schichten-Messdatensatzes und der Aufnahme seiner zugehörigen Referenzdaten keine Messdaten von Schichten aufgenommen werden, welche nicht in den mindestens zwei Schichten des Schichten-Messdatensatzes umfasst sind,
- Erstellen von Kalibrierungsdaten aus dem vollständigen Datensatz,
- Trennen des Schichten-Messdatensatz in Einzelschicht-Messdatensätze der simultan manipulierten Schichten unter Verwendung der Kalibrierungsdaten,
- Rekonstruieren von Bilddaten aus den Einzelschicht-Messdatensätzen.

2. Verfahren nach Anspruch 1, wobei das Rekonstruieren der Bilddaten eine Vervollständigung der Einzelschicht-Messdatensätze unter Verwendung der Kalibrierungsdaten umfasst.

3. Verfahren nach einem der vorgehenden Ansprüche, wobei alle Echosignale eines Schichten-Messdatensatzes nach einem einzigen Multi-Band-RF-Anregungspuls aufgenommen werden.

4. Verfahren nach einem der vorhergehenden Ansprüche, wobei die Aufnahme der Magnetresonanzdaten gemäß einem CAIPIRINHA-Verfahren erfolgt.

5. Verfahren nach einem der vorhergehenden Ansprüche, wobei die Aufnahme der Magnetresonanzdaten gemäß einer Turbo-Spinecho-Sequenz, insbesondere gemäß einer HASTE-Sequenz, erfolgt.

6. Verfahren nach einem der vorhergehenden Ansprüche, wobei die Referenzdaten verschachtelt mit den Schichten-Messdaten aufgenommen werden.

7. Verfahren nach einem der Ansprüche 1 bis 5, wobei die Referenzdaten im Anschluss an die Aufnahme der Schichten-Messdaten aufgenommen werden.

8. Verfahren nach Anspruch 7, wobei die Referenzdaten nach Einstrahlen eines zweiten Multi-Band-RF-Pulses zur simultanen und selektiven Manipulation der mindestens zwei Schichten als Schichten-Referenzdaten aufgenommen werden.

9. Verfahren nach Anspruch 8, wobei die Schichten-Referenzdaten in Referenzdaten getrennt werden, die jeweils nur Referenzdaten einer der mindestens zwei Schichten umfassen.

10. Verfahren nach Anspruch 7, wobei die Referenzdaten nach Einstrahlen von Einzelschicht-RF-Anregungspulsen aufgenommen werden.

11. Magnetresonanzanlage (1) umfassend, eine Magneteinheit (3), eine Gradienteneinheit (5), eine Hochfrequenzeinheit (7) und eine Steuereinrichtung (9) mit einer Hochfrequenz-Sende-/Empfangs-Steuerung (7') mit einer Multi-Band-RF-Pulseinheit (7a), wobei die Steuereinrichtung (9) dazu ausgebildet ist, ein Verfahren nach einem der Ansprüche 1 bis 10 auf der Magnetresonanzanlage (1) auszuführen.

12. Computerprogramm, welches direkt in einen Speicher einer Steuereinrichtung (9) einer Magnetresonanzanlage (1) ladbar ist, mit Programm-Mitteln, um die Schritte des Verfahrens nach einem der Ansprüche 1 bis 10 auszuführen, wenn das Programm in der Steuereinrichtung (9) der Magnetresonanzanlage (1) ausgeführt wird.

13. Elektronisch lesbarer Datenträger mit darauf gespeicherten elektronisch lesbaren Steuerinformationen, welche zumindest ein Computerprogramm nach Anspruch 12 umfassen und derart ausgestaltet sind, dass sie bei Verwendung des Datenträgers in einer Steuereinrichtung (9) einer Magnetresonanzanlage (1) ein Verfahren nach einem der Ansprüche 1 bis 10 durchführen.
